# EUROPEAN PATENT APPLICATION

(11) **EP 0 995 502 A2**
(43) Date of publication of application: **26.04.2000**
(21) Application number: 99307919.3
(22) Date of filing: 07.10.1999
(51) Int. Cl.: B09B 3/00, A61G 9/00, A61L 11/00

(54) **Process for disposing of human wastes, container for collecting human wastes and container-grinding machine**

(30) Priority: 08.10.1998 AR 1005027
(71) Applicant: Toia, Mario Fernando, 1425 Capital Federal, Buenos Aires (AR)
(72) Inventor: Toia, Mario Fernando, 1425 Capital Federal, Buenos Aires (AR)
(74) Representative: Daniels, Jeffrey Nicholas

(57) **Abstract**

A process for disposing of human wastes in an efficient and environmentally or pathologically safe manner, a grindable container for collecting human wastes and a crushing machine for said containers. The container is made of a grindable, preferably biodegradable material such as cellulose pulp, being sterilized and delivered preferably enclosed in polyethylene or polypropylene bags. The process begins by withdrawing the container from the bag and employing it in a conventional manner. The container is then bagged again, preferably in a sealed paper bag. The bagged container is then transferred, together with its contents, to a crushing machine where it is milled, disinfected, deodorized and finally liquefied so as to be readily poured into the sewer system, without causing a deleterious ecological impact on the environment. The container is designed to hold human wastes without any leaks or cracks and the crushing machine is designed to be used at any health-care or rest-home center.

## Description

The present invention relates to a process for disposing of human wastes, a container for collecting human wastes and a container-grinding machine for carrying out such process.

The importance of the sanitary disposal of human wastes from health-care centers has been a constant issue in every community, and this importance keeps growing as long as hospitals and clinics tend to improve their health and prophylactic standards in view of the great prevalence of high-risk diseases.

The main interest of urban authorities is not only targeted to avoid sewerage system overloading, but also to guarantee that these are capable of delivering organic wastes without contaminating the environment, since they may represent a growth medium for contagious diseases and for insects that convey such diseases.

Known processes for collecting and recovering human wastes from health-care centers are limited to the use of plastic or metal containers, which are presumably easy to clean, for transferring wastes to a nearby collecting sewer-inlet. After being used, it is the responsibility of the hospital staff to appropriately clean and disinfect said containers. These procedures have proven to be ineffective and diseases spreading means throughout treatment centers and rest homes. It is apparent that this requirement has not been met because of the lack of elements that could overcome the above problems in an effective and cost-effective manner.

It is therefore an overall object of this invention to overcome the problem of the lack of hygiene and asepsis of conventional human waste collection methods in health-care centers and rest homes.

### OBJECTS OF THE INVENTION

It is an object of the present invention to provide a process for collecting and transporting human wastes in a sanitary and safe manner, without involving the contact of personnel with these wastes, enclosing them in a disposable container, for the purpose of processing said container in a grinding machine and discharging the resulting fluid into the sewer-pipe.

It is another object of the present invention to provide a disposable, grindable, and substantially biodegradable container that is useful to carry out said process.

It is a further object of the present invention to provide a disposable, grindable, container that is built of a substantially non-biodegradable material and useful to carry out said process.

It is a further object of the present invention to provide a sterile and packaged container, which is disposable, grindable, and substantially biodegradable, and useful to carry out the above process.

It is still a further object of the present invention to provide a sterile and packaged container, which is disposable, grindable, and built of a substantially non-biodegradable material, that is useful to carry out said process.

It is another object of the present invention to provide a grinding machine or crushing mill to carry out the above process.

### DESCRIPTION OF THE DRAWINGS

Figure 1 shows a flow chart of the process as disclosed in the present invention.

Figure 2 shows a front view of the crushing mill used in the process of the present invention.

Figure 3 shows a side view of the crushing mill used in the process of the present invention.

Figure 4 is a plan view that shows the location of the pieces of equipment used in the first step of the process for making disposable containers, in a pulp embodiment.

Figure 5 is a plan view that shows the location of the pieces of equipment used in the second step of the process for making disposable containers, in a pulp embodiment.

Figure 6A is a side view of the disposable container embodiment in the shape of a bed urinal for male patients.

Figure 6B is a plan view of the disposable container embodiment in the shape of a bed urinal for male patients.

Figure 7A is a side view of the disposable container embodiment in the shape of a flat bed-pot.

Figure 7B is a plan view of the disposable container embodiment in the shape of a flat bed-pot.

Figure 8 is a side view of the alternative, non-disposable handle on a 1:2 scale.

Figure 9A is a side view of a framed, non-disposable handling device, on a 1:5 scale.

Figure 9B is a plan view of a framed, non-disposable handling device, on a 1:5 scale.

Figure 10 shows the flow chart of process for manufacturing disposable containers in a pulp embodiment.

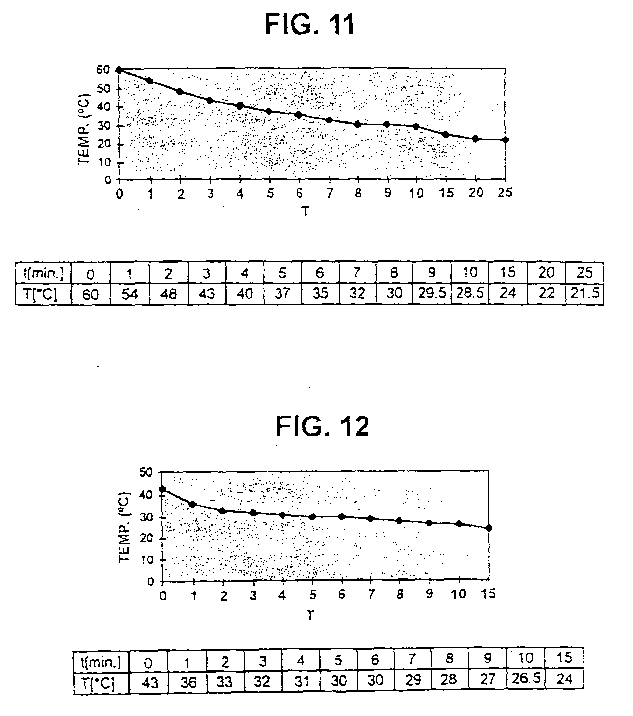

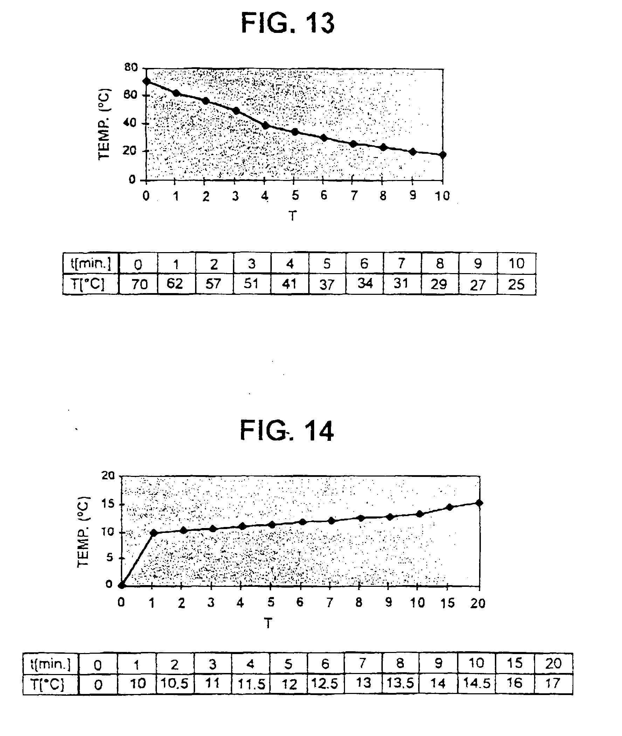

Figures 11 - 14 are temperature charts = *f* (time) for permeability essays described later.

### DESCRIPTION OF THE PREFERRED EMBODIMENT

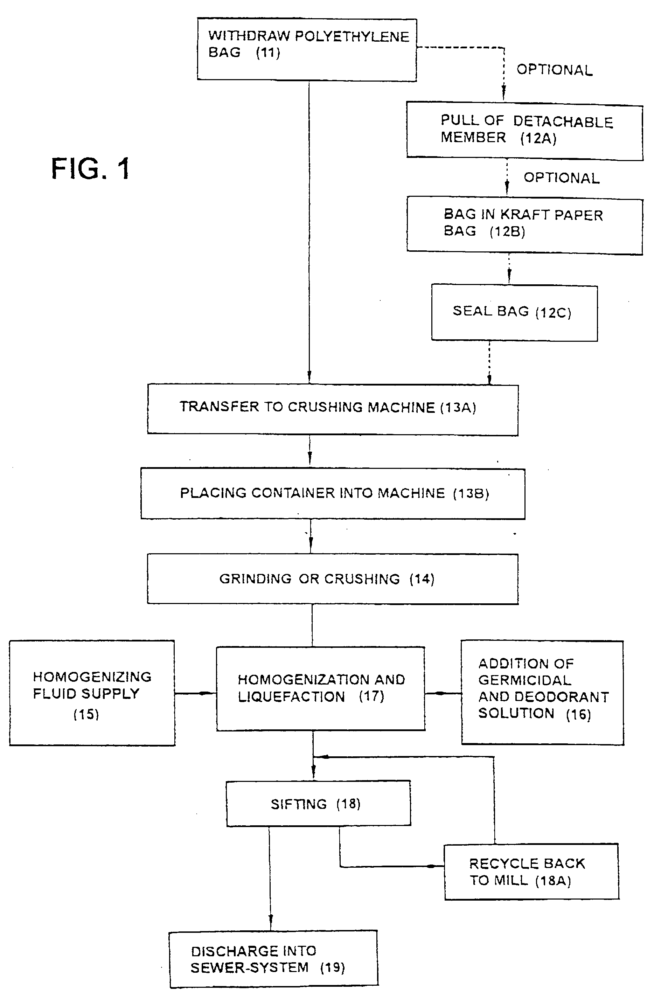

The present invention refers to a process for disposing of wastes from people that are unable to care for themselves, whether it comprises feces, urine. menstrual fluids or any other organic material emitted by human beings, which require a safe handling and guaranteed disinfection. It also relates to the collection container employed and to the grinding or crushing machine used to crush said container. The method shown in Figure 1 relies on the use of collection containers such as flat chamber pots, bed urinals for male patients, etc., that are made of a disposable, grindable and preferably substantially biodegradable material which does not contaminate the environment when being delivered to its final destination. When mentioning the disposable character of the material it is meant that it is a very low cost material. This will allow for the advantage of using it only once, which insures the complete sanitation of the process. This latter condition will lead the person responsible for cleaning the health care center to dispose of the container after it has been used, and said person will not even consider the possibility of reusing said container, because that will not bring about any economic profit, thus insuring the appropriate performance of the sanitary procedure, even in those places where there are no systematic means of control over the personnel.

In a highly preferred embodiment, the material comprising said disposable, grindable and substantially biodegradable container is cellulose pulp. In an alternative embodiment, the material proposed comprises any other cellulose-derived product, since these are grindable and low cost materials, e.g. cardboard, cork-like agglomerate, etc. These are alternative materials since they are not considered substantially biodegradable.

The process for collecting human wastes in sanitary containers that are built of a disposable material insures the use thereof by only one person and only once. Such collection containers are lighter and easier to handle by users on account of the material they are made of and, in every case, the material the disposable containers are built of will be grindable, i.e., it will be capable of being crushed.

The present invention provides: a process for disposing of human wastes, comprising the steps of: collecting human wastes in a container; transferring said container, together with the wastes it contains, into a crushing mill; crushing said container, together with its contents, using said crushing mill; providing a homogenizing fluid for the wastes to be crushed; homogenizing and liquefying the mass; sifting said mass through a mesh or sieve; recycling the pieces retained in the sieve back to the crushing mill; and discharging the end product preferably into the sewer system.

In a preferred embodiment the present invention provides a process for disposing of human wastes, characterized by comprising the steps of: withdrawing the package from the container immediately before use; collecting human wastes into said container; transferring said container after it is used and delivering it, together with the wastes it contains, into a crushing mill based on rollers and rotary blades; crushing said containers, together with their contents, by said crushing mill; providing a homogenizing fluid for the mass to be crushed; adding a germicide and deodorant solution to said mass; homogenizing and liquefying the mass; sifting said mass through a mesh or sieve; recycling the pieces retained in the sieve back to the crushing mill and discharging the end product into the sewer system.

### PROCESS FOR MANUFACTURING DISPOSABLE CONTAINERS

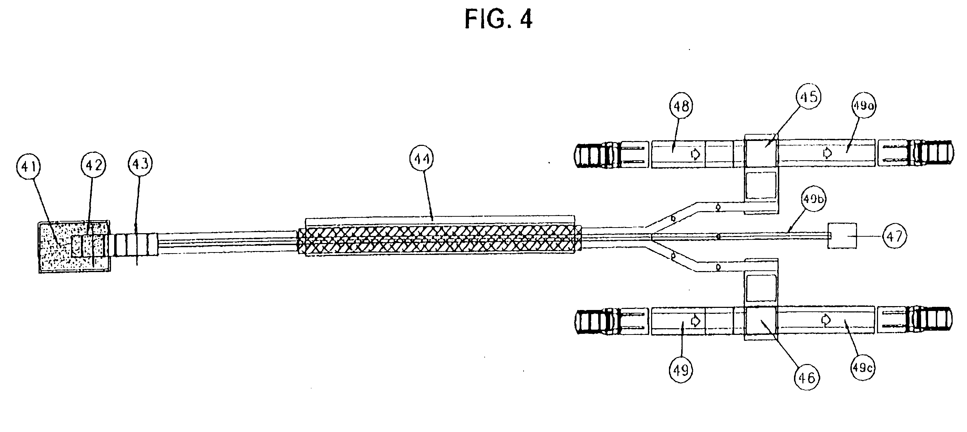

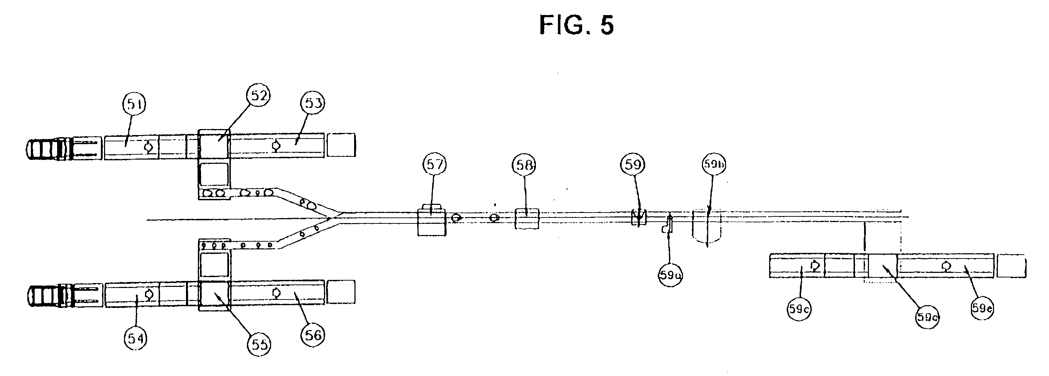

The process for manufacturing the above containers will depend on the selected material and does not pertain to this invention, insofar as it is known in the art as a process for forming cellulose pulp or other cellulose-derived materials into such containers. In the instance of the preferred embodiment, wherein the material chosen comprises cellulose pulp, Figures 4 and 5 show a known process for manufacturing molded pulp.

The reference numbers in Figure 4 are:
- 41.: Paste bat
- 42.: First roller
- 43.: Second roller
- 44.: Dryer oven
- 45.: Lower portion palletizer or containers themselves
- 46.: Lid palletizer
- 47.: Handle collector.
- 48.: Flat-link chain feeder of lower portion holders (stackable).
- 49.: Flat-link chain feeder of lid holders (stackable)
- 49A.: Lower portion holder conveyor.
- 49B.: Handle conveyor belt.
- 49C.: Lid holder conveyor belt.

Figure 4 shows a primary production line assembly into which the cellulose pulp enters and which provides the three main portions of the disposable container: the lower portion of the container or container itself, the handle and the lid.

The references in Fig. 5 are:
- 51.: Conveyor for the lower portions of the container.
- 52.: Depalletizer for the container lower portions.
- 53.: Empty container conveyor.
- 54.: Lid holder conveyor.
- 55.: Lid depalletizer.
- 56.: Empty container conveyor.
- 57.: Assembly - binding device for binding lower portions to their lids.
- 58.: Handle positioning device.
- 59.: Scented pad inserting device.
- 59A.: Inkjet decoder.
- 59B.: Packaging station.
- 59C: Empty pallet feeder.
- 59D.: Case packaging - palletizing device
- 59E.: Conveyor for pallets with cases.

Figure 5 shows a secondary production line assembly into which the three members from the line disclosed in Figure 4 will enter, said members exiting as fully assembled and packaged containers. Figure 5 shows an adhesive-laying and assembling machine to bind the lower portions to their respective lids. It is a pneumatic device that receives container lids from a delivery means fed by a lid holder through a conveyor belt and a feeding hopper.

Thereafter, and upon retention of the lower part of the disposable container itself, coming from a parallel line fed by container holders, the lid of the container, which has previously been provided with a biodegradable adhesive, is placed on the lower container and assembled onto it.

In the same Figure 5 a handle-inserting device is shown. Handles are fed through a vibrating/ turning hopper, which arranges the position of the handles by placing them so that they move downward in a descent hopper that is registered with the feeding line. The adhesive-laying and final placing of the handle is carried out by means of an electropneumatic device driven by a PLC (programmable loop controller).

It is important to point out that, in a highly preferred embodiment, the disposable container is delivered packed into a sealed polyethylene or polypropylene bag, so as to keep its asepsis and sterilization, before being used.

In the step of baking the pulp, the temperature to which the material is subjected is 130°C, whereby any bacteria present therein, as well as other contaminating agents, are destroyed. Thereafter, being a target to maintain the asepsis of the container until it is bagged for delivery, the whole production line is installed within a cleanness-control chamber, in accordance with "clean room" techniques, so as to achieve a "gray" area scoring, with a particle count of less than 10 µg/m³, using the pollution measuring standards according to ANSI/ ASHRAE 62 - 1989. The packaging of the resulting disposable container is carried out into the polyethylene or polypropylene bags in a final step before they leave the enclosure. The bagged assemblies are then irradiated with UV light to insure their asepsis during storage prior to use, and bags are heat-sealed after the containers are placed therein.

### DETAILED DESCRIPTION OF THE PROCESS OF THE INVENTION

The process of the invention is based on a sequence of steps that must be strictly pursued in order to insure the sanitary status of the institution it is carried out in.

It is worth stating that the process relies on simple grinding and liquefying devices through which a homogeneous and liquid mass is obtained as an end product, which allows for its transfer into the sewer system.

The process in Figure 1 starts with a step comprising withdrawing (11) the container out of the polyethylene, polypropylene or other similar materials , prior to use, which is then disposed of in a conventional manner that does not pertain to the process of the invention. Thereafter, the collection of human wastes is effected (12) into the disposable container in a conventional manner. In an optional embodiment, which involves the use of detachable handles or detachable, framed handle assemblies, an optional step (12A) of detaching said optional additional members is considered after the collecting step (12). In a further preferred embodiment. After that, a further step of bagging (12B) the container is performed by putting the container with its waste contents into a *Kraft-type*-type paper disposable bag that is provided with a coated inner side to make it impervious to splashes or spills, and closing the open end so as to prevent unpleasant odors to spread out into the environment. In any of the bag-sealing procedures, an element for preserving the biodegradability of the assembly is used. In a preferred embodiment, a glued area with a biodegradable adhesive is used, or else the operator may alternatively use a cotton string to strangle the open end of the bag. The string should be made of cotton so as to keep the overall biodregadability of the product.

The container is then transferred (13A) together with its contents, with or without a Kraft-type paper bag, to a grinding machine or a roller and blade mill. The time it takes to transfer a disposable container to the grinding machine should be less than 2 hours, being this time dependable on the number of grinding machines available in the building. For example, if the boarding floor of a hospital or clinic has a considerable number of wards (over 15), it is desirable to have at least one crushing equipment in every floor and a transferring cart per floor. However, the optimum number of grinding machines and transferring carts required per floor will vary according to the average number of hospitalized people with ambulatory impairments or difficulties.

The disposable containers are then delivered (13B) into the machine through the inlet thereof and are subjected to milling or grinding (14) by the mentioned rollers. At the inlet there is a perforated pipe (14), which supplies homogenizing fluid, preferably water, from several nozzles suitably arranged so as to help each disposable container slip into the grinding area, lubricate the milling operation and wash the feeding duct.

In the next step, proceeding with the flow chart in Fig. 1, after the disposable container is ground or crushed (14), the homogenizing and liquefaction step begins (17) through a cut and stir effect provided by high speed spinning of rotating blades. The entire mass of the material comprising the end product of this process is then sifted (18) by forcing the mass to pass through a mesh or sieve to prevent big residues from being pushed on to the following step. The sifted fragments fall by gravity into the lower area wherefrom the resulting fluid is discharged (19) into the sewer system, preferably by gravity and alternatively by means of a sewage pump.

At the time the mill is started, a spray-nozzle (atomizer) adds a germicide-deodorant solution to the product (15). In a preferred embodiment this solution is bleach (sodium hypoclorite). In alternative embodiments, other solutions may be used, such as concentrated sodium hypoclorite (commercially available as "liquid chlorine"), chloroxylenol parachlorometoxylenol, chlorometaxylenol, quaternary ammonium compounds, or benzalkonium chloride.

The fluid that is added in the homogenizing fluid addition step of the homogenizing and liquefaction process (17) is preferably water, due to its low cost and ready supply. The volume added in every step of the process is about 10 liters ( aprox. 2.5 gallons), supplied from the water line of the building, wherefore a connection for the machine is provided with a water blocking-deblocking element. preferably a solenoid valve wired 110 VCA ( 60 Hz) or a motorized ball valve. The addition (16) of a dilute germicidal solution to the ground mass is effected from an ancillary vessel, preferably from an elevated "backpack" type reservoir, mounted on the outside of the device and continuously refilled from the tap-water feeding line through a known mechanical means comprising a valve and float. The germicidal solution is added to the reservoir water so as to insure that the entire crushed product is disinfected, to disinfect the inner members of the machine and avoid the growth of fungi and mold. The addition of the concentrated germicidal solution is carried out by means of a known inverted container drop by drop system commonly used in toilet reservoirs.

The crushing mill is enclosed in a soundproof chamber that preferably comprises a double wall covering filled with a soundproof material so as to minimize the noise resulting from the device, particularly in a residential or therapeutic recovering environment. Preferably, the soundproof material will comprise glass wool or expanded polyurethane in the shape of sheets with a thickness of preferably 0.75 - 1 inch , over the walls, the bottom and top sides of the chamber. The rest of the machine is made of parts and materials that are well known in the art.

### PREFERRED EMBODIMENT OF THE DISPOSABLE CONTAINER

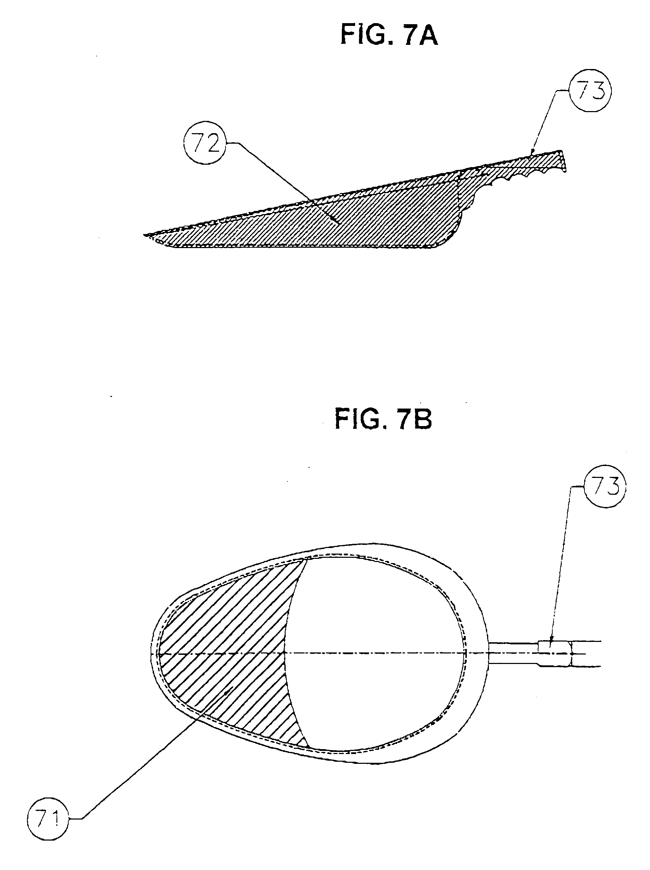

Fig. 7 shows a preferred embodiment, wherein both the lid (71) and the handle (73) are integral parts of the lower portion (72) of the disposable container, and this embodiment requires about 200 to 300 g ( 0.440- 0.660 lbs) of cellulose pulp or other alternative cellulose-derived material.

### ALTERNATIVE EMBODIMENTS OF THE DISPOSABLE CONTAINER

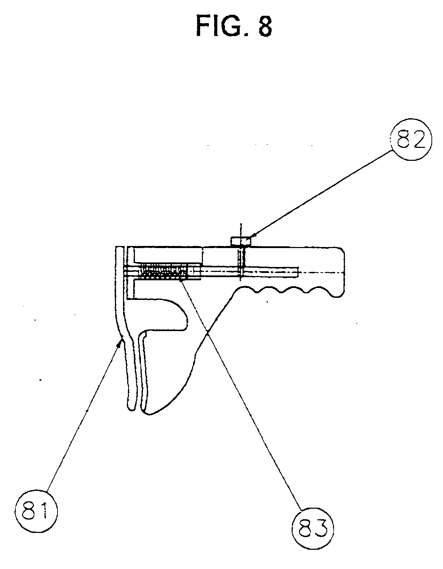

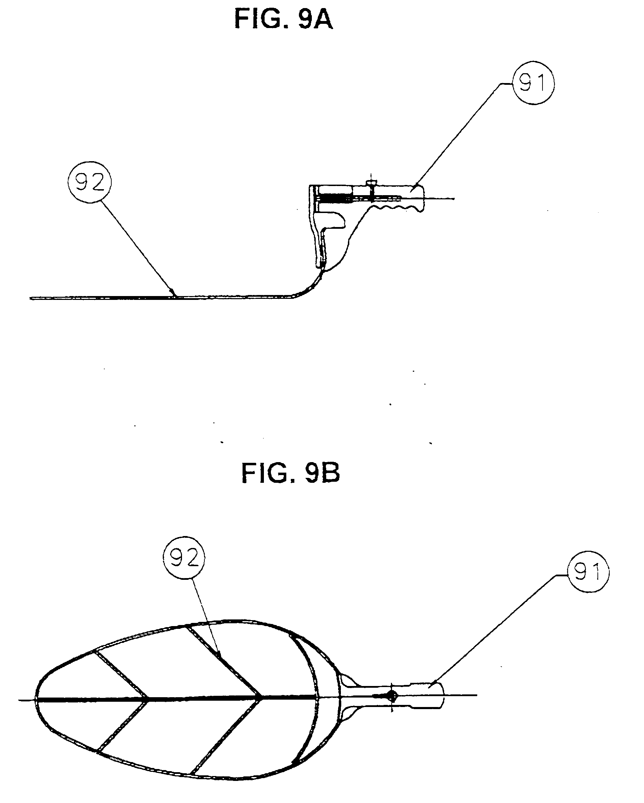

In other alternative embodiments, the handle is not a part of the container, its use being thus dictated by the requirements of the production line. In these instances, three alternative embodiments are envisioned:
A) Both the container and the lid are made of pulp and the handle comprises a detachable member (Fig. 8) that is detached in step (12A) of the process. In this optional embodiment the detachable handle may be made, e.g., of plastics, stainless steel, prepainted iron plate, aluminum, bakelite or ferrous or non-ferrous alloys.
   Figure 8 shows the following elements:
   - 81.: Expandable clamp
   - 82.: Driving cam for the expandable clamp.
   - 83.: Compression spring.
B) Identical to item (A) with the exception that the handle is a part of a handle-frame assembly (Figs. 9A and 9B) that serves the purpose of handling the container for its transfer. The following elements are shown in Figs. 9A and 9B:
   - 91.: Detachable handle.
   - 92.: support frame
C) The use of other devices or pieces that are not part of the disposable container that might serve as handlers and are made of the same or different materials such as: plastic, stainless steel, prepainted iron plate, aluminum. bakelite or ferrous or non-ferrous alloys.

According to the above, the elements described in items "A", "B" and "C" are reusable and must be detached (12A) from the disposable container before placing it in the Kraft-type paper bag. The use of these reusable elements may be desirable, since they allow to lower the cost of the forming process and a lesser amount of raw materials is required since they avoid the need to mold the handle.

### DETAILED DESCRIPTION OF THE CRUSHING MILL

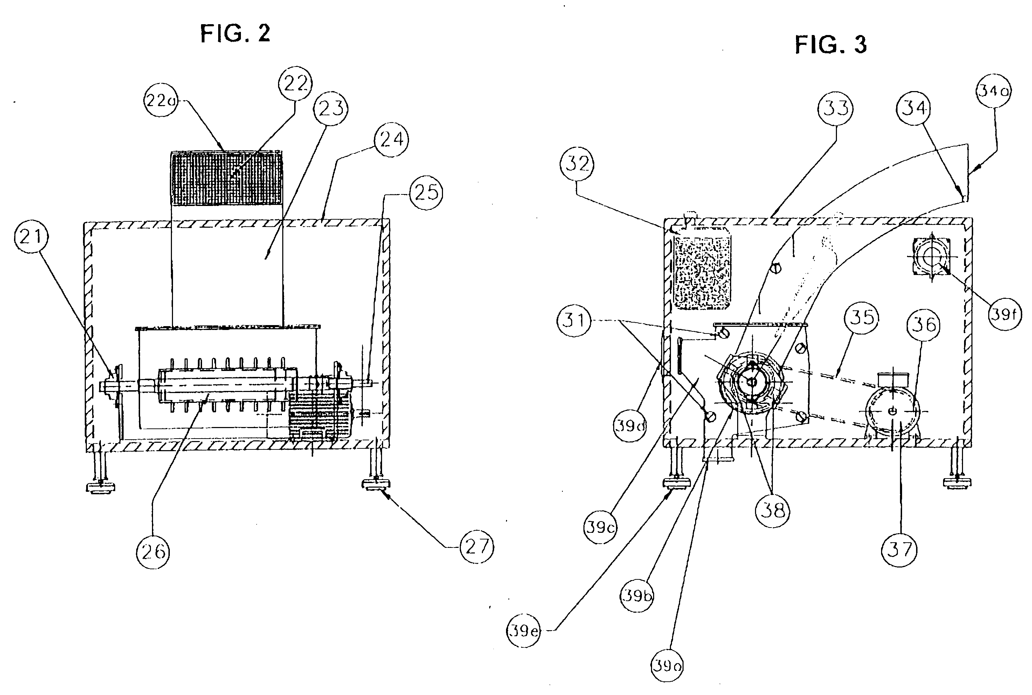

Fig. 2 is a front view of the crushing mill, with the following reference numbers:
- 21.: Self-centering and self-sealing bearings on the central shaft
- 22.: Retractable lid with sealed closure
- 23.: Stainless steel hopper.
- 24.: Soundproof coating.
- 25.: Drive shaft.
- 26.: Rotary mill

Fig. 3 shows a side view of the machine, having the following reference numbers:
- 31.: Water and germicide spray nozzles inside the crushing chamber.
- 32.: Flask containing germicidal solution
- 33.: Stainless steel housing
- 34.: Water spray perforated pipe.
- 34A.: Container inlet.
- 35.: Driving belts
- 36.: Driving pulley.
- 37: Motor.
- 38.: Stationary mills.
- 39A.: Outside connection to sewer system.
- 39B.: Filtering mesh.
- 39C.: Crushing stainless steel chamber.
- 39D.: Manhole cover inlet to the crushing chamber.
- 39E.: Germicidal solution dose pump.
- 39F.: Antivibration supports for the machine.

In a preferred embodiment, the machine is provided with a PLC to execute the automation of the machine's operation and to control all the functions thereof, by receiving signals from end-of-stroke sensors in order to emit, by means of a luminescent-sound operation signal, status signals and failure interruption alarm (not shown in the drawings). In order to keep the cost of the machine as low as possible, the use of a PLC with 4 digital inputs (plus 2 for future use), 3 analog inputs and 8 digital outputs, in order to receive on/off signals from 4 end of stroke sensors has been contemplated. The following failures would thus be detected: a) lack of water in the reservoir, b) mobile mill not running (belt cut or slipping), c) outlet to sewer clogged and d) engine thermal detector actuated by overload. In a preferred embodiment, but without being limited to any specific model or brand, the use of a Honeywell® model DCP 100 PLC, with Honeywell® - Microswitch® sensors, BZ/BA type and Honeywell® flow switches, model FS4 have been suggested. Programming of the automatic functions of the grinding or crushing machine are not a part of the invention and, in a preferred embodiment, this is carried out in a "ladder" programming, or in any language recommended by the PLC supplier.

In a preferred embodiment, a wash and inner disinfecting dose pump, as well as antivibration supports, to eliminate vibrations that might be transmitted to the floor and/or walls, are also included.

### TESTS

A detailed description of the tests carried out on the materials used in prototypes is now shown:

Materials: The test container prototypes were made of the following materials:
A) Molded pulp (MP).
B) Molded cardboard (single and double corrugation).

### A) Molded pulp (MP):

Cellulose pulp is a cellulose paste prepared from a mixture of fresh raw materials and recycled material from paper, cardboard, pieces of cloth, etc., from vegetable, organic sources with the addition of chemical or natural substances, plasticizers or binders, and dye pigments or bleaches. This material is known to be used in the manufacture of boxes and trays (so called "maples") for egg packaging, pizza package boxes and similar food products.

### A-1) Determination of the specific weight per area unit and density of the material (average values)

Specific Weight per area unit: Usually expressed in g/m².
Density: Expressed in kg/dm³.

### Procedure:

Four Templates of equal dimensions are cut with 5 cm x 5 cm = 25 cm² (i.e. aprox. 3,9 sq. inches) and weighed on a DPSA brand analytical scale, model 8009 P, with 5 ½ digit resolution, maximum capacity 1 kg, lowermost graduation 0,1 g and precision ± 1 %.

Once the samples are taken the following values are obtained:
1. 2.1443 g
2. 2.1565 g
3. 2.1188 g
4. 2.1541 g

**Obtained Values :**
- Specific Weight:: 857 g/m². ( 0.553 g/ sq. inch)
- Density:: 0.857 kg/dm³ ( 14.0 g / cu. inch)

### A-2) Determination of longitudinal and transverse tear strength (ELMENDORF test)

Test apparatus: ELMENDORF (empirical unit in reference scale)

This test is used for paper, cardboard and cellulose-derived materials. The harder the material, the lower the resulting ELMENDORF number.

### Test Methodology:

1) Five samples of the material are cut and held with two clamps.
2) The bladed pendulum is released from its upper position, and in its stroke the blades cut the test sample, dragging the reference indicator, which stops, thus indicating an empirical non-dimensional value on the Elmendorf scale.
3) The test is repeated with another batch of samples after which the obtained values are averaged.
   - Pulp test Results: longitudinal tear:: 40 - 50 Elmendorf
   - Transverse tear:: 65 - 75 Elmendorf

### A-3) Determination of stress-strain and flexural strength upon the use of pressure (bursting):

This is effected by placing the sample between two ring-shaped clamps and exerting hydraulic pressure through a membrane until either stress or crushing causes the rupture.

The obtained result was: 150 - 200 ibs/sq. inch.

### A-4) Permeability tests

Several containers in the shape of a truncated cone, having a diameter of 33 mm ± 0,5 mm ( 1.3 inches ± 0.02 ) across the major base, 5 mm ± 0,5 mm ( 0.2 inches ± 0.02 )across the minor base and a height of 20 mm ± 0,5 mm ( 0.78 inches ± 0.02) were used. Permeability tests on liquids at various initial temperatures were carried out by subjecting the pulp material to the action of water at a level of 7 to 15 mm ( 0.27 - 0.59 inches) over the bottom surface.

The following instruments were used for these tests:
(1) Wet bulb thermometer (Range -10°C to 110°C)
(1) 10 ml measuring pipette.

Material tested: plate with areas in the shape of truncated cones.

The temperature variations were measured as a function of time, working at room temperature (R.T. = 20 °C), and examining the product's permeability.

### Test 1:

- Liquid:: Water
- Volume:: 5 ml.
- H_{liquid}:: 7 mm ( 0.27 inches)
- Ti=: 60°C
- R.T.=: 21°C

Variations in the temperature of water for the duration of the test are shown in Fig. 7.

*Results:* No permeation of humidity was observed.

### Test 2:

- Liquid:: Water
- Volume:: 10 ml.
- H_{liquid}:: 15 mm
- Ti=: 43°C
- R.T.=: 21°C

The change in the temperature of water for the duration of the test is shown in Fig. 8.

*Results:* No permeation of humidity was observed.

### Test 3:

- Liquid:: Water
- Volume:: 10 ml.
- H_{liquid}:: 12 mm
- Ti=: 70°C
- R.T. =: 21°C

The change in the temperature of water for the duration of the test is shown in Fig. 9.

*Results:* Some wetness was observed on the walls of the test material.

### Test 4:

- Liquid:: Water
- Volume:: 10 ml.
- H_{liquid}:: 15 mm
- Ti=: 0°C
- R.T.=: 21°C

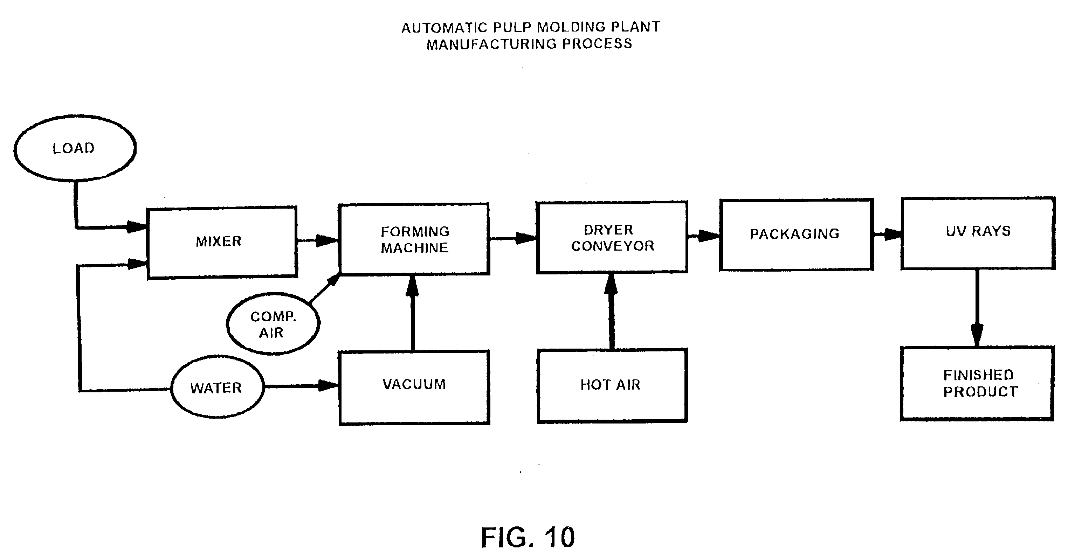

The change in the temperature of water for the duration of the test is shown in Fig. 10.

*Results:* No leakage was observed.

### Overall Results:

- Permeability of the pulp is a reverse function to the temperature of the liquid.
- When the temperature of the liquid used is not over 60°C the material retains its imperviousness without impairment, for a minimum time of 2 hours.
- From the tests performed, the only instance wherein humidity was observed on the walls of the material was in Test 3 and this is believed to be due to the high initial temperature of the liquid (70°C). It is worth noting that human waste liquids are usually at a normal initial temperature of between Ti 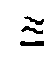 37°C and Ti 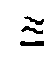 42°C and that, except for highly unusual circumstances, they are not expected to reach the container at a temperature outside this range. Therefore, the temperatures used in the above assays are believed to reflect extreme and more adverse conditions that usual. In addition, the fact that the initial temperature of wastes tends to promptly reach the room temperature favors the imperviousness of the material.

### B) Molded cardboard ( MC).

This material is stress-molded cardboard produced under conditions of humidity, temperature and pressure exerted by eccentric-shaft presses, pneumatic presses, electrohydraulic presses or quick presses.

It is a commercially available material, used for building package boxes and other common uses. Depending on the strength that is required, there are two different configurations used for boxes: simple corrugation (low strength) or double corrugation (high strength).

### B-1) Determination of specific weight and density of the material

These assays were not performed because it is a material of common commercial use, with values being published by the suppliers.

### B-2) Determination of longitudinal and transverse tear strength

(ELMENDORF test): These tests were not performed because it is a material of common commercial use.

### B-3) Determination of stress-strain and flexural strength upon application of pressure (bursting):

No test was performed. The following values were achieved from tables published by suppliers.
Simple corrugation: 40 - 50 lbs/sq. inch
Double corrugation 180 - 200 lbs/sq. inch

### B-4) Permeability Tests

These tests were not performed because it is a material of common commercial use.

### Mechanical strength tests performed on the disposable container made of cellulose pulp.

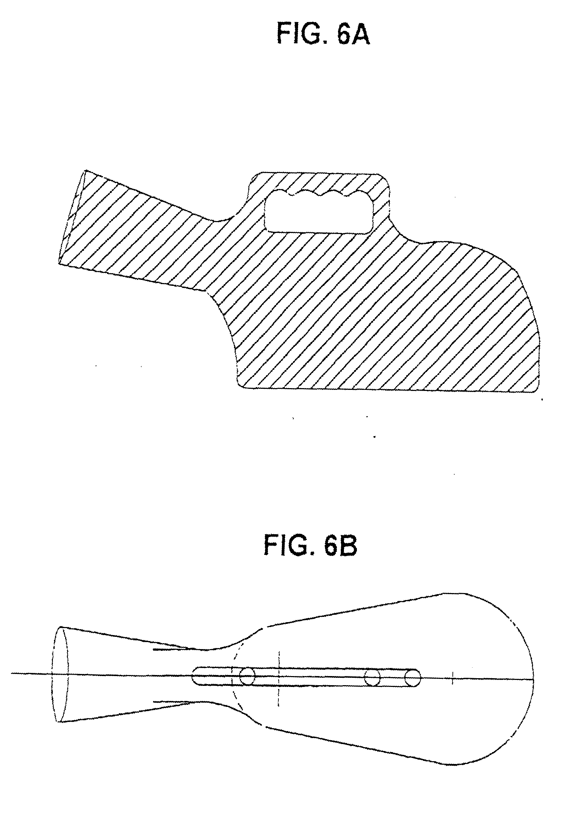

Flat bed-pot prototypes were made of cellulose pulp (Fig. 6B), with the insertion of nerves or ribs to prevent the product from collapsing under stress loads higher than the maximum weight of 150 kg pertaining to a human being sitting over it under conditions of use.

A weight of 150 kg was considered to be equivalent to a person sitting on the flat bed-pot, spreading some of his or her legs and buttocks' weight outside the same (mattress, side pillows, chairs, floor, etc.). The weight used is higher than the average weight of a person and it also takes into account the additional stress from the dynamic application of the load, for the user will bear on the container with a certain drive. The force was applied by means of a fixed hand press and it was monitored by means of a dynamometer. The application time was of 30 minutes, non-stop.

Load test results: On performing the test, its stress rigidity was considered as satisfactory. When exposed to loads, slight plastic strains were observed, which did not preclude the use of the container, retaining the volume thereof as a liquid receptor on tilting the flat bed-pot during handling. No cracks, ruptures or tears in the material were detected , the container thus retaining its impervious character.

### Rib arrangement:

In a preferred embodiment, the insertion of reinforcing ribs is considered in order to insure the structural rigidity of the container upon impacts and maximum use. The ribs
- may or may not be aligned with the symmetric axis of the product;
- may be a part of the contour in a radial arrangement that restricts the volume thereof;
- plugs may be added in the area of the liquid receiving pail, their arrangement remaining parallel to the volumetric boundaries; or
- may be placed inside or outside, and of the same material or one that is easy to crush or biodegradable.

### Use of deodorant additives in the disposable container:

In a preferred embodiment it is desirable to use deodorant products that do not imply a considerable increase in cost but which do upgrade the product. Pads (multilayer material soaked in scented substances) are used in the polyethylene or polypropylene preserving bags comprising the aseptic packaging of the product. The pad must be stable until use; it may preferably comprise a removable label that peels off on withdrawing the preserving bag, with the scent or deodorant remaining in the adhesive thereof.

### Experiments for testing the crushing of the container.

These were carried out on an experimental model of crushing machine. The following results were achieved:
Number of containers broken down per minute = 3 to 5.
Amount of wash water used per cycle = 5 to 10 I.
Amount of germicide and/or disinfectant used per cycle = 10 ml.
Motor power = 2 to 3 HP
Particulate material = 10 to 12 mm mesh.

### Precautions taken during transfer of containers:

In a preferred embodiment, it is contemplated to effect the collection of containers used in clinics, hospital wards, nursing homes, etc., with the help of a transfer carriage wherein the containers are carefully stored, preferably within their preserving bag until they are crushed by the crushing mill and optionally without using such bag.

### Features of the transfer carriage:

1. Minimum gage, about 600 mm ( 23.6 inches)
2. Smooth rolling (wheels with polyurethane treads and bands)
3. Stainless steel plate housing with container storage areas.
4. It should not have areas and/or sectors which make cleaning and disinfection difficult.
5. Sliding door openings to facilitate their opening in narrow hallways.
6. Drawbar on side of the two turning wheels.

## Claims

1. A process for disposing of human wastes, comprising the steps of:
collecting human wastes in a container;
transferring said container, together with the wastes it contains, into a crushing mill;
crushing said container, together with its contents, using said crushing mill;
providing a homogenizing fluid for the wastes to be crushed;
homogenizing and liquefying the mass;
sifting said mass through a mesh or sieve;
recycling the pieces retained in the sieve back to the crushing mill; and
discharging the end product.

2. A process according to claim 1, wherein said container is made of a disposable and grindable material.

3. A process according to claim 1 or claim 2, wherein said container is made of a substantially biodegradable material, which does not contaminate the environment.

4. A process according to claim 1 or claim 2, wherein said container is made of a substantially non-biodegradable material.

5. A process according to any preceding claim, wherein said container comprises a bag, and before the bag is transferred to the crushing mill, the bag is sealed.

6. A process according to claim 5, wherein said bag is made of Kraft-type paper.

7. A process according to claim 1, comprising a further step of pulling off a non-disposable fastening means, which is bound to said container, after the step of collecting wastes.

8. A process according to any preceding claim, wherein the homogenizing fluid comprises water.

9. A process according to any preceding claim, wherein the homogenizing fluid comprises water, and a germicidal agent and a deodorant agent in solution, wherein the solution is in the same mixture proportions, or in different mixture proportions, as the one directly applied to the crushed mass.

10. A process according to claim 9, wherein the germicidal agent and/or the deodorant agent comprises dilute sodium hypoclorite.

11. A process according to claim 9, wherein the germicidal agent and/or the deodorant agent comprises concentrated sodium hypoclorite, or parachlorometaxylenol, chlorometaxylenol, quaternary ammonium or benzalkonium chloride compounds.

12. A container for collecting human wastes, made of a disposable and grindable material, to be used by one patient only, a single time.

13. A container according to claim 12, being made of a substantially biodegradable material, which will not contaminate the environment.

14. A container according to claim 13, wherein said biodegradable material comprises cellulose pulp.

15. A container according to claim 14, wherein said material substantially retains its imperviousness to human waste fluids for up to 2 hours provided the initial temperture thereof is not over 60°C (140°F).

16. A container according to claim 12, said material being substantially non-biodegradable.

17. A container according to claim 16, wherein said material comprises a cellulose-derived material comprising cardboard, reconstituted cork-type material or a similar material.

18. A container according to any of claims 12-17, comprising a lid, a lower portion and a handle.

19. A container according to any of claims 12-18, comprising a lid, a lower portion and a detachable fastening member.

20. A container according to claim 19, wherein said detachable fastening member comprises a removable handle.

21. A container according to claim 19 or claim 20, wherein said detachable fastening member comprises an expandable clamp, an actuating cam for the expandable clamp and a compression spring.

22. A container according to any of claims 19-21, wherein said detachable fastening member comprises a detachable handle-frame assembly.

23. A container according to any of claims 19-22, wherein said detachable fastening member is made of plastics, stainless steel, prepainted iron plate, aluminium, bakelite, or ferrous or non-ferrous alloys.

24. A container according to any of claims 12-23, which is capable of withstanding a maximum static weight of 150 kg (330 lbs), applied in a downward vertical direction.

25. A container according to any of claims 12-24, which is packed inside a polyethylene or polypropylene bag, under asepsis and sterilization conditions achieved through the application of UV light during its manufacture, which takes place in an environment-controlled room.

26. A container crushing machine, comprising a housing, containing a motor for driving, by means of a pulley and belt assembly, a set of rotary blades enclosed within a crushing chamber, a set of stationary blades for complementing the rotary blades thus forming a crushing arrangement; an inlet to a stainless steel hopper leading to the crushing chamber; a filtering mesh capable of sifting the milled pieces of the container; a reservoir for containing germicidal and deodorant fluid fed by a tap-water inlet, provided with a mechanical stopper and a tap-water inlet feeding a perforated pipe equipped with spray-nozzles capable o spraying the interior of the machine.

27. A crushing machine according to claim 26, wherein said housing is soundproof housing.

28. A crushing machine according to claim 27, wherein soundproofing is by means of double fiber walls with glass wool or expanded polyurethane thereinbetween.

29. A crushing machine according to any of claims 26-28, wherein said germicidal and deodorant fluid reservoir contains a water-diluted solution of a concentrated germicidal and deodorant liquid, said liquid being supplied by gravity and dropwise to said reservoir.
